# EUROPÄISCHE PATENTSCHRIFT

(11) **EP 3 202 236 B1**
(45) Veröffentlichungstag und Bekanntmachung des Hinweises auf die Patenterteilung: **25.07.2018**
(21) Anmeldenummer: 15787567.5
(22) Anmeldetag: 28.10.2015
(51) Int. Cl.: H05B 37/02, A61B 90/35, H05B 33/08, H05B 35/00, F21W 131/20

(54) **OPERATIONSLEUCHTE UND VERFAHREN ZUM BETREIBEN EINER OPERATIONSLEUCHTE**
SURGICAL LIGHT AND METHOD FOR OPERATING A SURGICAL LIGHT
SCIALYTIQUE ET PROCÉDÉ POUR FAIRE FONCTIONNER UN SCIALYTIQUE

(30) Priorität: 07.11.2014 DE 102014222793
(43) Veröffentlichungstag der Anmeldung: 09.08.2017
(73) Patentinhaber: TRUMPF Medizin Systeme GmbH + Co. KG, 07318 Saalfeld (DE)
(72) Erfinder: SCHMID, Michael, 88433 Schemmerhofen (DE); FRENZEL, Mathias, 82110 Germering (DE)
(74) Vertreter: Prüfer & Partner mbB Patentanwälte · Rechtsanwälte
(86) Internationale Anmeldenummer: PCT/EP2015/074995
(87) Internationale Veröffentlichungsnummer: WO 2016/071178

(56) Entgegenhaltungen:
- EP-A1- 1 722 157
- US-A1- 2008 285 820
- US-A1- 2010 243 823

## Beschreibung

Die Erfindung betrifft eine Operationsleuchte und ein Verfahren zum Betreiben einer Operationsleuchte, im Speziellen eine Operationsleuchte mit zusätzlichen Funktionalitäten und ein Verfahren zum Ausleuchten eines Operationsfelds an einem menschlichen Körper mit dieser Operationsleuchte.

Es sind Operationsleuchten bekannt, die neben der Ausleuchtung des Operationsfelds zusätzliche Funktionalitäten aufweisen. Beispielsweise sind Operationsleuchten mit andockbaren Kamerasystemen ausgerüstet. Ein Anbringen einer Kamera beispielsweise an einem Leuchtenkörper der Operationsleuchte wird durch die Operationsleuchte erkannt. Die Kamera wird mit elektrischer Energie versorgt und kann über Bedienelemente der Operationsleuchte gesteuert werden. Beispielsweise kann die Kamera für eine Bildausrichtung gedreht, oder der Bildausschnitt kann über eine Zoom-Funktion verkleinert oder vergrößert werden.

Aus der Druckschrift DE 20 2007 007 054 U1 und aus der entsprechenden Druckschrift US2008/0285820 A1 ist ferner eine Operationsleuchte bekannt, bei der eine Farbtemperatur des ausgestrahlten Lichts eingestellt werden kann. Die Information über die Farbtemperatur des eingestellten Lichts wird über die Steuerungsvorrichtung der Operationsleuchte an eine Kamera an der Operationsleuchte weitergegeben, so dass ein Weißabgleich der Kamera mittels der Farbtemperaturinformation ausgeführt werden kann.

Die Druckschrift EP 1 785 665 A1 zeigt eine Operationsleuchte, bei der Beleuchtungsmodule des Leuchtenkörpers ausgewechselt werden können. Die Beleuchtungsmodule werden werksseitig bezüglich der Farbtemperatur und der Intensität des ausgestrahlten Lichts für verschiedene Betriebszustände kalibriert, wobei Kalibrierwerte in den Beleuchtungsmodulen abgespeichert werden. Diese Kalibrierwerte werden von einer zentralen Steuerung der Operationsleuchte aus den Beleuchtungsmodulen abgerufen und die Beleuchtungsmodule werden durch die zentrale Steuerung der Operationsleuchte entsprechend ihrer Kalibrierwerte betrieben.

Es sind also Operationsleuchten bekannt, die Funktionsmodule, nämlich Module, die eine bestimmte Funktion ausführen und die an die Operationsleuchte andockbar sind, aufweisen und die Datenverbindungen mit den Funktionsmodulen aufweisen, so dass diese Funktionsmodule entsprechend ihrer Eigenschaften und Funktionsmöglichkeiten betrieben werden.

Wenn aber ein spezielles Funktionsmodul in Verbindung mit der Operationsleuchte betrieben wird, bei dem nicht nur der Betrieb des speziellen Funktionsmoduls sondern der gesamten Leuchte beeinflusst wird, muss die Operationsleuchte durch einen serviceseitigen Eingriff für die Befestigung des Funktionsmoduls und für dessen Ansteuerung durch die Operationsleuchte angepasst werden.

Der Erfindung liegt die Aufgabe zugrunde, eine Operationsleuchte zu schaffen, die flexibel und ohne großen Aufwand an den Betrieb von speziellen Funktionsmodulen, die den Betrieb der Operationsleuchte beeinflussen, angepasst werden kann.

Die Aufgabe wird durch eine Operationsleuchte gemäß Anspruch 1 und ein Verfahren gemäß Anspruch 14 gelöst.

Durch ein Vorsehen einer Schnittstelle an einem Leuchtenkörper einer Operationsleuchte und an einem separaten Funktionsmodul und durch eine Anpassung einer Steuerungsvorrichtung der Operationsleuchte, so dass der Leuchtenkörper mit seinem Leuchtmittel und ggf. weiteren Funktionsbauteilen entsprechend abgespeicherter Betriebsdaten, die aus dem separaten Funktionsmodul ausgelesenen werden, betrieben wird, ist es möglich, die Operationsleuchte flexibel ohne service-seitige Eingriffe an den Betrieb von speziellen nachträglich vorgesehenen Funktionsmodulen anzupassen.

Die Erfindung wird anhand von Ausführungsbeispielen unter Bezugnahme auf die beigefügten Zeichnungen erläutert.

Insbesondere zeigen
- Fig. 1: einen Leuchtenkörper einer Operationsleuchte mit separaten Funktionsmodulen; und
- Fig. 2: einen vergrößerten Ausschnitt des Leuchtenkörpers von Fig. 1 mit einem der separaten Funktionsmodule und einer Aussparung dafür, und einer schematisierten Darstellung der ersten und zweiten Schnittstelle.

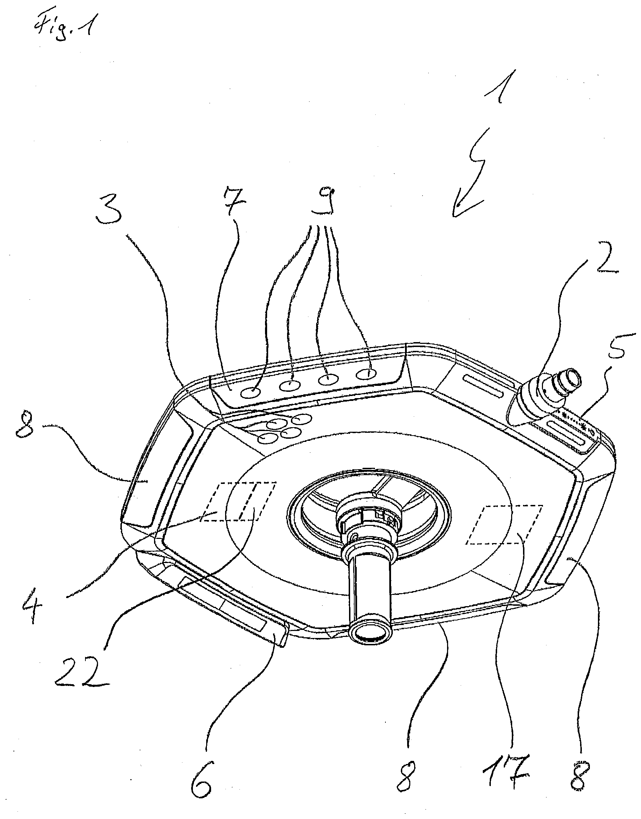

Fig. 1 zeigt einen Leuchtenkörper 1 einer Operationsleuchte. Der Leuchtenkörper 1 ist über ein Drehgelenk 2 mit einer nicht gezeigten Aufhängeeinrichtung beispielsweise an einer Raumdecke in allen Richtungen schwenkbar befestigt.

Der Leuchtenkörper 1 ist mit mehreren Leuchtmitteln 3 versehen. Die Leuchtmittel 3 befinden sich innerhalb des Leuchtenkörpers 1 und strahlen in der in Fig. 1 gezeigten Ausrichtung des Leuchtenkörpers 1 Licht nach unten aus, um ein Operationsfeld an einem menschlichen Körper auszuleuchten. Von den Leuchtmitteln 3 sind hier nur vier von einer Mehrzahl dargestellt. Die Leuchtmittel 3 sind hier als LEDs ausgeführt und im Wesentlichen über eine gesamte Lichtaustrittsfläche am Leuchtenkörper 1 verteilt. Alternativ ist zumindest ein einziges Leuchtmittel vorhanden.

Die Operationsleuchte weist ferner im Leuchtenkörper 1, oder alternativ an einer anderen geeigneten Stelle, beispielsweise an einer Deckenbefestigung, eine Steuerungsvorrichtung 4 auf.

Die Steuerungsvorrichtung 4 weist einen Speicherbereich 22 auf, in dem Betriebsdaten gespeichert sind. Insbesondere können optional später beschriebene Beleuchtungsszenarien darin gespeichert sein.

Die Operationsleuchte ist mit Bedienelementen 5, hier am Leuchtenkörper 1 versehen, wobei die Bedienelemente 5 auch an der Aufhängeeinrichtung oder einem nicht gezeigten Wandpanel vorgesehen sein können.

Die Leuchtmittel 3 sowie die Bedienelemente 5 sind mit der Steuerungsvorrichtung 4 verbunden. Die Steuerungsvorrichtung 4 steuert die Leuchtmittel 3 entsprechend Einstellungen an den Bedienelementen 5. Durch die Bedienelemente 5 ist ein Einstellen einer Intensität des von den Leuchtmitteln 3 ausgestrahlten Lichts, also einer Helligkeit in dem Operationsfeld, sowie eines Durchmessers eines durch die Leuchtmittel 3 in dem Operationsfeld erzeugten Leuchtfelds möglich. Optional sind weitere Einstellmöglichkeiten, beispielsweise eine Farbtemperatur des ausgestrahlten Lichts, möglich.

Der Leuchtenkörper 1 ist an zwei Andockpunkten mit separaten Funktionsmodulen 6, 7 versehen. Die Funktionsmodule 6, 7 sind hier im Außenbereich, also am Umfang des Leuchtenkörpers 1 angeordnet. Alternativ können die Funktionsmodule 6, 7 auch, in Abhängigkeit von ihrer Funktion, an einer anderen Stelle am Leuchtenkörper 1, beispielsweise an der Oberseite oder in der Lichtaustrittsfläche, vorgesehen sein.

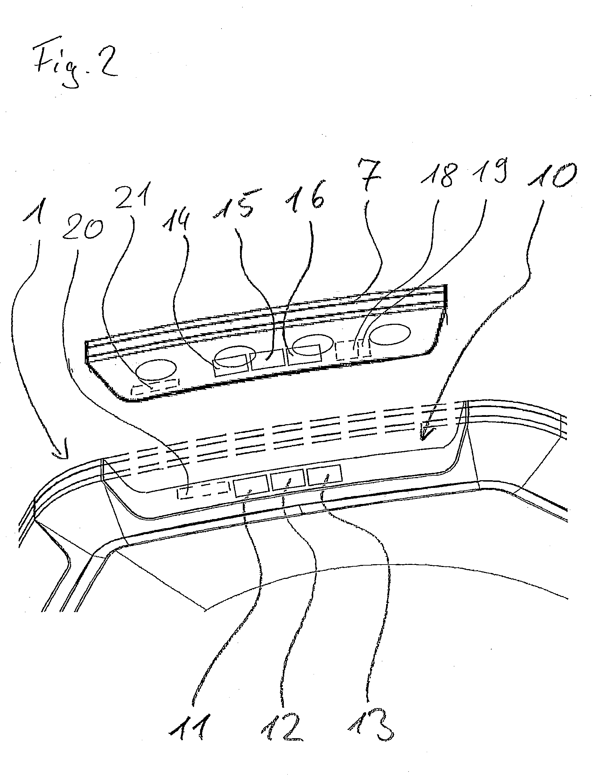

Der Leuchtenkörper 1 ist aber noch mit weiteren Andockpunkten mit Aussparungen 10 (siehe Fig. 2) versehen, die, wenn keine Funktionsmodule 6, 7 angebracht sind, mit Abdeckungen 8 abgedeckt sind, um hygienische Verhältnisse nicht zu verschlechtern. Insbesondere wird so eine aerodynamische Form des Leuchtenkörpers 1 ermöglicht, auch wenn keine Funktionsmodule 6, 7 am Leuchtenkörper 1 angebracht sind. Somit wird ein Strömungsfeld einer Lüftungsdecke über der Operationsstelle kaum beeinträchtigt.

Das Funktionsmodul 6 ist hier ein Sensormodul, mit dem der Abstand zwischen dem Leuchtenkörper 1 und dem Operationsfeld gemessen werden kann. Das Sensormodul 6 ist mit der Steuerungsvorrichtung 4 verbunden und überträgt erfasste Daten an die Steuerungsvorrichtung 4, die basierend darauf beispielsweise einen Leuchtfelddurchmesser oder eine Helligkeit anpasst bzw. konstant hält.

Das Funktionsmodul 7 ist hier ein Lichtmodul. In dem Lichtmodul 7 sind weitere Leuchtmittel 9 vorgesehen. Die Leuchtmittel 9 in dem Lichtmodul 7 verändern die lichttechnischen Eigenschaften der Operationsleuchte. In dem Lichtmodul 7 sind weitere Leuchtmittel 9 vorgesehen, die hinsichtlich ihrer lichttechnischen Eigenschaften, beispielsweise einer Farbtemperatur oder einer Intensität des ausgestrahlten Lichts, mit den Leuchtmitteln 3 innerhalb des Leuchtenkörpers 1 vergleichbar sind. Damit ist möglich, die Intensität des ausgestrahlten Lichts unter Einbehaltung der normativen Anforderungen zu erhöhen. Weiterhin ist es alternativ auch möglich, beispielsweise den Leuchtfelddurchmesser zu vergrößern. Optional wird auch ein verbessertes Schattenmanagement, also ein Unterleuchten von Hindernissen oder Ausblenden von schattenbildenden Leuchtmitteln, bereitgestellt.

Alternativ können auch andere oder spezielle Leuchtmittel 9 mit anderen lichttechnischen Eigenschaften vorgesehen sein, um beispielsweise eine Fluoreszenzdiagnostik durchzuführen oder eine andere Farbtemperatur des ausgestrahlten Lichts einstellen zu können.

In weiteren Alternativen sind auch beispielsweise Kameramodule oder Sensormodule als Funktionsmodule möglich. In einer Ausführungsform ist ein einziges Funktionsmodul vorgesehen, es können aber auch mehrere Funktionsmodule vorgesehen sein. Zudem sind alternativ Kombinationen von Funktionen, wie etwa eine Integration von Leuchtmitteln und Sensorik, in einem Funktionsmodul möglich.

Die Funktionsmodule sind so geformt, dass sie im Wesentlichen an eine Grundform des Leuchtenkörpers angepasst sind, um die aerodynamische Form des Leuchtenkörpers nicht zu beinträchtigen.

In Fig. 2 ist ein vergrößerter Ausschnitt des Leuchtenkörpers 1 mit einer Aussparung 10 für eines der separaten Funktionsmodule 6, 7 und einer schematisierten Darstellung der ersten und zweiten Schnittstelle gezeigt.

Die Aussparung 10 ist ebenfalls lediglich schematisch gezeigt. Grundsätzlich bietet die Aussparung 10 einen der Andockpunkte und Raum für das Andocken eines Funktionsmoduls 6, 7.

In der Aussparung 10 am Leuchtenkörper 1, hier also im Außenbereich des Leuchtenkörpers 1, ist eine erste Schnittstelle vorgesehen, die eine erste Befestigungseinrichtung 11, einen ersten Stromversorgungsanschluss 12 und eine erste Datenverbindung 13, die hier schematisch gezeigt sind, aufweist. Der Stromversorgungsanschluss 12 kann optional auch in der Datenverbindung 13 integriert sein. In dem Leuchtenkörper 1 sind hier fünf identische erste Schnittstellen im Außenbereich des Leuchtenkörpers 1 vorgesehen. Alternativ kann nur eine erste Schnittstelle oder eine andere Anzahl vorgesehen sein. Die ersten Schnittstellen sind jeweils identisch ausgeführt, so dass eine Standardschnittstelle ausgebildet wird.

Das Funktionsmodul 7 weist eine aus einer zweiten Befestigungseinrichtung 14, einem zweiten Stromversorgungsanschluss 15 und einer zweiten Datenverbindung 16, die hier ebenfalls schematisch gezeigt sind, bestehende zweite Schnittstelle auf. Die zweite Schnittstelle am Funktionsmodul ist mit der ersten Schnittstelle am Leuchtenkörper 1 verbindbar und dazu kompatibel.

Die erste Befestigungseinrichtung 11 und die zweite Befestigungseinrichtung 14 fixieren das Funktionsmodul 6, 7 an dem Leuchtenkörper 1. Sie sind optional so ausgestaltet, dass die erste und zweite Schnittstelle von Hand, also werkzeuglos verbunden und getrennt werden können, um eine Montage und Demontage möglichst einfach zu gestalten. Aus Sicherheitsgründen ist optional eine von Hand lösbare Verriegelung der Befestigungseinrichtungen 11, 14 vorgesehen. Alternativ ist auch die Montage oder Demontage nur mit Hilfe von Werkzeug möglich, um eine unberechtigte Montage oder Demontage zu verhindern.

Der erste Stromversorgungsanschluss 12 ist mit einer Stromversorgungseinheit 17 der Operationsleuchte verbunden. Über den ersten Stromversorgungsanschluss 12 oder optional über die erste Datenverbindung 13, die dann mit der Stromversorgungseinheit 17 verbunden ist, und dem zweiten Stromversorgungsanschluss 15 oder optional der zweiten Datenverbindung 16 an dem Funktionsmodul 6, 7 wird dieses mit Energie versorgt.

Die erste Datenverbindung 13 am Leuchtenkörper 1 ist mit der Steuerungsvorrichtung 4 verbunden. Die zweite Datenverbindung 16 am Funktionsmodul 6, 7 ist mit einem Speicherelement 18 im Funktionsmodul 6, 7 verbunden. Optional ist auch eine Steuerungseinrichtung 19 zum Ansteuern des Funktionsmoduls darin vorgesehen. Die Verbindung kann jeweils über Kabel oder alternativ auch kabellos, beispielsweise über Funk, erfolgen.

In einer alternativen Ausführungsform können die ersten und/oder zweiten Schnittstellen auch unterschiedlich ausgeführt sein.

Sind beispielsweise erste Funktionsmodule 6, 7 nur an vorbestimmten Positionen an dem Leuchtenkörper 1 vorgesehen, angedockt zu werden, können die ersten Schnittstellen dementsprechend ausgebildet sein, dass nur die ersten Funktionsmodule 6, 7 mit einer bestimmten Ausprägung der zweiten Schnittstelle an den vorbestimmten Positionen am Leuchtenkörper 1 angedockt werden können. Die erste Schnittstelle und die zweite Schnittstelle sind dann beispielsweise über einen Vorsprung in einer der ersten oder zweiten Schnittstelle beispielsweise an einer vorbestimmten Position oder mit einer vorbestimmten Form und einer dazu komplementären Aussparung in der anderen der ersten oder zweiten Schnittstelle kodiert. Weitere Möglichkeiten zur Kodierung sind bestimmte Ausprägungen, wie beispielsweise der Form oder der Position, der ersten und zweiten Befestigungseinrichtung 11, 14, des ersten und zweiten Stromversorgungsanschlusses 12, 15, oder der ersten und zweiten Datenverbindung 13, 16.

Sind andere als die ersten Funktionsmodule 6, 7 an anderen vorbestimmten Positionen oder an sämtlichen Positionen vorgesehen, andockbar zu sein, sind die jeweiligen ersten und zweiten Schnittstellen, beispielsweise wie oben beschrieben, dementsprechend kodiert.

In dem Leuchtenkörper 1 ist eine Einrichtung vorhanden, durch die ein Typ des angedockten Funktionsmoduls 6, 7 erkannt werden kann. Der Typ wird beispielsweise durch die Steuerungsvorrichtung 4 erkannt, indem bestimmte Signale aus dem Funktionsmodul 6, 7 ausgewertet werden. Alternativ sind Schalter oder Sensoren vorhanden, die bestimmte Merkmale, beispielsweise Vorsprünge, von verschiedenen Typen von Funktionsmodulen 6, 7 erkennen. Die Steuerungsvorrichtung 4 steuert die Leuchtmittel 3 im Leuchtenkörper 1 und gegebenenfalls das Funktionsmodul 6, 7 entsprechend dessen Typ.

In dem Leuchtenkörper 1 ist ferner optional für jeweils eine der ersten Schnittstellen eine Schalteinrichtung 20 für den ersten Stromversorgungsanschluss 12 und/oder die erste Datenverbindung 13 vorgesehen. Die Schalteinrichtung 20 verbindet im eingeschalteten Zustand den ersten Stromversorgunganschluss 12 mit der Stromversorgungseinheit 17 und die erste Datenverbindung 13 mit der Steuerungsvorrichtung 4 der Operationsleuchte und unterbricht im ausgeschalteten Zustand die Verbindungen.

Sofern der Leuchtenkörper 1 mit den optionalen Schalteinrichtungen 20 versehen ist, sind die Funktionsmodule 6, 7 jeweils mit einer Aktivierungseinrichtung 21 versehen. Die Aktivierungseinrichtung 21 schaltet die an der Aussparung 10 angeordnete Schalteinrichtung 20 ein, wenn das Funktionsmodul 6, 7 über die erste Schnittstelle und die zweite Schnittstelle in der jeweiligen Aussparung 10 an dem Leuchtenkörper 1 befestigt ist. Wenn das jeweilige Funktionsmodul 6, 7 von dem Leuchtenkörper 1 getrennt wird, also nicht mehr befestigt ist, wird auch die Verbindung zwischen dem ersten Stromversorgungsanschluss 12 und der Stromversorgungseinheit 17, und zwischen der ersten Datenverbindung 13 und der Steuerungsvorrichtung 4 der Operationsleuchte getrennt.

Die Schalteinrichtung 20 ist hier ein Reedkontakt im Leuchtenkörper 1 und die Aktivierungseinrichtung 21 ist ein Magnet in dem Funktionsmodul 6, 7, der den Reedkontakt schaltet. Alternativ sind auch andere Schalt- bzw. Aktivierungseinrichtungen möglich, wie beispielsweise ein mechanisch betätigbarer Schalter am Leuchtenkörper 1 und eine Schaltfläche am Funktionsmodul 6, 7.

In einer alternativen Ausführungsform ist ein separater Schalter vorhanden, der den ersten Stromverbindungsanschluss 12 mit der Stromversorgungseinheit 17 und die erste Datenverbindung 13 mit der Steuerungsvorrichtung 4 der Operationsleuchte verbindet und im ausgeschalteten Zustand unterbricht. Der separate Schalter ist entweder manuell oder über die Steuerungsvorrichtung 4 schaltbar.

In dem Speicherelement 18 des Funktionsmoduls 6, 7 sind Betriebsdaten des Funktionsmoduls 6, 7 gespeichert. Die Betriebsdaten sind im Fall eines Lichtmoduls 7 Betriebsparameter als Kalibrierwerte der Leuchtmittel 9. Optional kann auch eine Betriebssoftware in dem Speicherbereich abgelegt sein. Ferner können optional auch mit dem Funktionsmodul 6, 7 ausführbare Beleuchtungsszenarien in dem Speicherelement 18 gespeichert sein.

In alternativen Ausführungsformen sind die Bedienelemente 5 als anpassbare Bedienoberfläche ausgeführt. Die Bedienoberfläche passt sich automatisch an die Konfiguration der Operationsleuchte mit den Funktionsmodulen an und zeigt nur den Leuchtenkörper 1 mit den vorhandenen Funktionsmodulen 6, 7 an. In einer der Ausführungsformen zeigt die Bedienoberfläche eine Maximalkonfiguration, wobei in Abhängigkeit von der tatsächlichen Konfiguration Funktionalitäten der Operationsleuchte einschließlich der Funktionsmodule gesperrt oder aktiviert sind.

Im Betrieb werden bei einem Anschließen eines der separaten Funktionsmodule 6, 7 an einer der Aussparungen 10 an dem Leuchtenkörper 1 die jeweilige erste Befestigungseinrichtung 11 mit der zweiten Befestigungseinrichtung 14, und, optional über die eingeschaltete Schalteinrichtung 20, der jeweilige erste Stromversorgungsanschluss 12 sowie die jeweilige erste Datenverbindung 13 mit dem zweiten Stromversorgungsanschluss 15 und der zweiten Datenverbindung 16 der zweiten Schnittstelle des Funktionsmoduls 6, 7 verbunden. Die Steuerungsvorrichtung erkennt mittels der Erkennungseinrichtung zum Erkennen des Typs des Funktionsmoduls dessen Typ. In Abhängigkeit von dem Typ des Funktionsmoduls steuert die Steuerungsvorrichtung 4 dann die Leuchtmittel 3 im Leuchtenkörper 1. Optional wird durch die Steuerungsvorrichtung 4 auch das Funktionsmodul 6, 7 entsprechend seinem Typ angesteuert. Wenn Beleuchtungsszenarien für diesen Typ des Funktionsmoduls 6, 7 in dem Speicherbereich 22 der Steuerungsvorrichtung 4 enthalten sind, werden die Leuchtmittel 3 und im Falle des angedockten Lichtmoduls 7 auch seine Leuchtmittel 9 entsprechend dem abgespeicherten Beleuchtungsszenario angesteuert.
Optional schickt die Steuerungsvorrichtung 4 eine elektronische Abfrage an das Speicherelement 18 des Funktionsmoduls 6, 7, worauf gegebenenfalls Daten zur Erkennung des Typs des Funktionsmoduls 6, 7 und Betriebsparameter von der Steuerungsvorrichtung 4 der Operationsleuchte ausgelesen werden. Als weitere Option werden in dem Speicherelement 18 gespeicherte Beleuchtungsszenarien abgerufen. Die Steuerungsvorrichtung 4 steuert und betreibt nun basierend auf den Betriebsparametern bzw. den Beleuchtungsszenarien sowohl die Leuchtmittel 3 des Leuchtenkörpers 1 als auch das gerade angedockte Funktionsmodul 6, 7 sowie gegebenenfalls weitere von der Steuervorrichtung 4 des Leuchtenkörpers 1 angesteuerte Einrichtungen der Operationsleuchte oder weitere Funktionsmodule 6, 7.

Als eine Option sind zur Ansteuerung der Leuchtmittel 3 die Beleuchtungsszenarien in der Steuerungsvorrichtung 4 gespeichert. So ist beispielsweise in Verbindung mit angedockten Lichtmodulen 7 ein Beleuchtungsszenario abgespeichert, in dem bei einem Anschluss eines zusätzlichen Lichtmoduls 7 beispielsweise die maximale Intensität der Leuchtmittel 3 innerhalb des Leuchtenkörpers 1 reduziert wird. Die maximale Intensität der Leuchtmittel 3 innerhalb des Leuchtenkörpers 1 und gegebenenfalls der zusätzlichen Lichtmodule 7 wird in Abhängigkeit von einer Art und/oder Anzahl der zusätzlichen Lichtmodule 7 reduziert, so dass eine maximal zulässige Beleuchtungsstärke in dem Operationsfeld nicht überschritten wird, also sowohl das Funktionsmodul als auch der Leuchtenkörper 1 entsprechend der in dem Funktionsmodul gespeicherten Betriebsdaten angesteuert wird.

Ein weiteres Beleuchtungsszenario ist optional in Verbindung mit einem angedockten Sensormodul 6 möglich. Das Sensormodul 6 gibt Daten bezüglich einer Position eines zwischen dem Leuchtenkörper 1 und dem Operationsfeld vorhandenen Hindernisses an die Steuerungsvorrichtung 4. In dem Speicherbereich 22 der Steuerungsvorrichtung 4 ist ein Beleuchtungsszenario abgespeichert, in dem diejenigen Leuchtmittel 3, in dessen Lichtstrahl das Hindernis vorhanden ist gedimmt oder ausgeschaltet werden, um Schatten auf dem Operationsfeld zu vermeiden oder verringern. Optional werden alle oder einige der verbleibenden Leuchtmittel 3 mit erhöhter Intensität betrieben. In einer weiteren optionalen Ausführungsform ist der Leuchtenkörper 1 zusätzlich mit Lichtmodulen 7 versehen, dessen Leuchtmittel 9 in einem weiteren Beleuchtungsszenario in diese Funktion der Schattenvermeidung oder -verringerung eingeschlossen sind. Somit werden also die zusätzlichen Lichtmodule 7 gemäß aus einem anderen Funktionsmodul 6, 7 ausgelesenen Betriebsdaten angesteuert.

Bei einem Anschluss eines Funktionsmoduls 6, 7 an den Leuchtenkörper 1, dessen Funktionalität der Steuerungsvorrichtung 4 so nicht in der in der Steuerungsvorrichtung (4) hinterlegt war, wird in der Steuerungsvorrichtung 4 ein Betriebssoftware-Update gemäß der in dem Speicherelement 18 des Funktionsmoduls 6, 7 abgespeicherten Betriebssoftware durchgeführt.

## Patentansprüche

1. Operationsleuchte zum Ausleuchten eines Operationsfelds an einem menschlichen Körper
mit einem Leuchtenkörper (1),
einer Steuerungsvorrichtung (4), und
mindestens einem separaten Funktionsmodul (6, 7),
wobei
der Leuchtenkörper (1) mindestens ein Leuchtmittel (3) zum Ausleuchten des Operationsfelds, und
mindestens eine erste Schnittstelle mit einer ersten Befestigungseinrichtung (11), einem Stromversorgungsanschluss (12) und einer Datenverbindung (13) aufweist, die Steuerungsvorrichtung (4) zum Ansteuern des mindestens einen Leuchtmittels (3) vorgesehen ist und mit der Datenverbindung (13) der mindestens einen ersten Schnittstelle für eine Datenübertragung verbunden ist, und
das mindestens eine Funktionsmodul (6, 7) eine zweite Schnittstelle mit einer zweiten Befestigungseinrichtung (14), einem zweiten Stromversorgungsanschluss (15) und/oder einer zweiten Datenverbindung (16) aufweist, die an die erste Schnittstelle andockbar ist, **dadurch gekennzeichnet, dass** der Leuchtenkörper (1) eine Erkennungseinrichtung zum Erkennen eines Typs eines angedockten Funktionsmoduls (6, 7) aufweist, und
die Steuerungsvorrichtung (4) angepasst ist, das mindestens eine Leuchtmittel (3) im Leuchtenkörper (1) in Abhängigkeit von dem Typ des angedockten Funktionsmodul (6, 7) anzusteuern.

2. Operationsleuchte gemäß Anspruch 1, wobei mehrere identische erste Schnittstellen am Leuchtenkörper (1) vorgesehen sind.

3. Operationsleuchte gemäß Anspruch 2, wobei mehrere Funktionsmodule (6, 7) vorgesehen sind.

4. Operationsleuchte gemäß Anspruch 2 oder 3, wobei die ersten Schnittstellen im Außenbereich des Leuchtenkörpers (1) angeordnet sind.

5. Operationsleuchte gemäß einem der vorangehenden Ansprüche, wobei die erste Schnittstelle und die zweite Schnittstelle so ausgebildet sind, dass deren Verbinden und Trennen werkzeuglos erfolgt.

6. Operationsleuchte gemäß einem der vorangehenden Ansprüche, wobei die Steuerungsvorrichtung (4) einen Speicherbereich (22) aufweist, und in dem Speicherbereich (22) verschiedene Beleuchtungsszenarien gespeichert sind.

7. Operationsleuchte gemäß Anspruch 6, wobei mindestens eines der Funktionsmodule (6, 7) ein Lichtmodul aufweist.

8. Operationsleuchte gemäß Anspruch 7, wobei Beleuchtungsszenarien Daten aufweisen, die das Einhalten eines Grenzwerts für eine maximale Beleuchtungsstärke im Operationsfeld in Abhängigkeit von der Art und/oder der Anzahl der Lichtmodule sicherstellen.

9. Operationsleuchte gemäß einem der Ansprüche 6 bis 8, wobei mindestens eines der Funktionsmodule (6, 7) ein Sensormodul aufweist.

10. Operationsleuchte gemäß Anspruch 9, wobei das Sensormodul dazu angepasst ist, Daten bezüglich einer Position eines zwischen dem Leuchtenkörper (1) und dem Operationsfeld vorhandenen Hindernisses an die Steuerungsvorrichtung (4) zu geben, und ein Beleuchtungsszenario abgespeichert ist, wonach die Leuchtmittel (3) in Abhängigkeit von der Position des Hindernisses angesteuert werden, so dass Leuchtmittel (3) mit einem Lichtstrahl, in dem das Hindernis liegt, gedimmt oder ausgeschaltet werden.

11. Operationsleuchte gemäß einem der vorangehenden Ansprüche, wobei das mindestens eine Funktionsmodul (6, 7) ein Speicherelement (18) zum Abspeichern von Betriebsdaten aufweist, und
wobei die Steuerungsvorrichtung (4) angepasst ist, die Betriebsdaten aus dem Speicherelement (18) des mindestens einen Funktionsmoduls (6, 7) auszulesen und sowohl das mindestens eine Funktionsmodul (6, 7) als auch den Leuchtenkörper (1) entsprechend den ausgelesenen Betriebsdaten anzusteuern.

12. Operationsleuchte gemäß Anspruch 11, wobei die Steuerungsvorrichtung (4) dazu angepasst ist, nach jedem Verbinden der ersten Schnittstelle und zweiten Schnittstelle, die Betriebsdaten aus dem Speicherbereich des verbundenen Funktionsmoduls auszulesen und die Operationsleuchte entsprechend den ausgelesenen Betriebsdaten anzusteuern.

13. Operationsleuchte gemäß Anspruch 11 oder 12, wobei die gespeicherten Betriebsdaten sowohl Betriebsparameter als auch Betriebssoftware aufweisen.

14. Operationsleuchte gemäß Anspruch 13, wobei die Steuerungsvorrichtung (4) dazu angepasst ist, zu erkennen ob ein Funktionsmodul (6, 7) mit einer vorbestimmten so nicht in der in der Steuerungsvorrichtung (4) hinterlegten Funktion zum ersten Mal mit der Steuerungsvorrichtung (4) verbunden ist, und ein Update der Betriebssoftware der Steuerungsvorrichtung (4) gemäß der in dem Funktionsmodul (6, 7) gespeicherten Betriebssoftware durchzuführen.

15. Operationsleuchte gemäß einem der Ansprüche 11 bis 14, wobei die Steuerungsvorrichtung (4) dazu angepasst ist, ein bestimmtes Funktionsmodul (6, 7) entsprechend aus einem der anderen als dem bestimmten Funktionsmodul (6, 7) ausgelesenen Betriebsdaten anzusteuern.

16. Operationsleuchte gemäß einem der vorangehenden Ansprüche, wobei der Leuchtenkörper (1) mindestens eine Aussparung (10) für die Anbringung der Funktionsmodule (6, 7) und eine Abdeckung (8) aufweist, und die mindestens eine Aussparung (10) mit der Abdeckung (8) abdeckbar ist, wenn kein Funktionsmodul (6, 7) angebracht ist.

17. Operationsleuchte gemäß einem der vorangehenden Ansprüche, wobei die Funktionsmodule (6, 7) eine Aktivierungseinrichtung (21) und der Leuchtenkörper (1) für jede erste Schnittstelle eine Schalteinrichtung (20) aufweist, die von der Aktivierungseinrichtung (21) geschaltet wird, so dass der Stromversorgungsanschluss (12) der ersten Schnittstelle mit einer Stromversorgungseinheit (17) und/oder der Datenverbindungsanschluss (13) der ersten Schnittstelle mit der Steuerungsvorrichtung (4) verbunden ist, wenn das Funktionsmodul (6, 7) über die erste Schnittstelle und zweite Schnittstelle an dem Leuchtenkörper (1) befestigt ist, und der Stromversorgungsanschluss (12) der ersten Schnittstelle von der Stromversorgungseinheit (17) und/oder der Datenverbindungsanschluss (13) der ersten Schnittstelle von der Steuerungsvorrichtung (4) getrennt ist, wenn das Funktionsmodul (6, 7) nicht über die erste Schnittstelle und die zweite Schnittstelle an dem Leuchtenkörper (1) befestigt ist.

18. Verfahren zum Betreiben einer Operationsleuchte gemäß einem der Ansprüche 1 bis 17, mit den Schritten
- Verbinden der zweiten Schnittstelle des mindestens einen Funktionsmoduls (6, 7) mit der ersten Schnittstelle des Leuchtenkörpers (1); **gekennzeichnet durch**:
- Erkennen des Typs des angedockten Funktionsmoduls (6, 7);
- Ansteuern des mindestens einen Leuchtmittels (3) in Abhängigkeit von dem Typ des angedockten Funktionsmoduls (6, 7).

19. Verfahren gemäß Anspruch 18 mit einer Operationsleuchte gemäß Anspruch 6, wobei das mindestens eine Leuchtmittel (3) in Abhängigkeit von dem Typ des angedockten Funktionsmoduls (6, 7) entsprechend einem abgespeicherten Beleuchtungsszenario angesteuert wird.

20. Verfahren gemäß Anspruch 18 oder 19 mit einer Operationsleuchte gemäß Anspruch 11, mit den zusätzliche Schritten
- Auslesen der Betriebsdaten aus dem Speicherelement (18) des mindestens einen Funktionsmoduls (6, 7);
- Ansteuern sowohl des Funktionsmoduls (6, 7) als auch des Leuchtenkörpers (1) entsprechend den ausgelesenen Betriebsdaten.

21. Verfahren gemäß einem der Ansprüche 18 bis 20 mit dem Schritt
- Ansteuern des Funktionsmoduls (6, 7) entsprechend den aus einem der anderen Funktionsmodule (6, 7) ausgelesenen Betriebsdaten.

22. Verfahren gemäß einem der Ansprüche 18 bis 21 mit einer Operationsleuchte gemäß Anspruch 17 mit dem zusätzlichen Schritt
- Schalten einer Schalteinrichtung (20) mittels einer Aktivierungseinrichtung (21) und dabei Verbinden des Stromversorgungsanschlusses (12) der ersten Schnittstelle mit der Stromversorgungseinheit (17) und Verbinden der Datenverbindung (13) der ersten Schnittstelle mit der Steuerungsvorrichtung (4).

23. Verfahren gemäß einem der Ansprüche 20 bis 22 mit einer Operationsleuchte gemäß Anspruch 15 mit dem zusätzlichen Schritt
- Ansteuern eines der Funktionsmodule (6, 7) entsprechend den aus einem der anderen Funktionsmodule (6, 7) ausgelesenen Betriebsdaten.

## Claims

1. Surgical lamp, for illuminating a surgical field on a human body, having
a lamp body (1),
a control device (4), and
at least one separate function module (6, 7),
wherein
the lamp body (1) comprises at least one illuminant (3) for illuminating the surgical field, and
at least one first interface having a first fixation device (11), a power supply terminal (12) and a data connection device (13),
the control device (4) is provided for controlling the at least one illuminant (3) and is connected to the data connection device (13) of the at least one first interface for data transmission, and
the at least one function module (6, 7) comprises a second interface having a second fixation device (14), a second power supply terminal (15) and/or a second data connection device (16) that can be docked to the first interface, **characterized in that**
the lamp body (1) comprises a recognition device for recognizing a type of a docked function module (6, 7), and
the control device (4) is configured to control the at least one illuminant (3) in the lamp body (1) depending on the type of the docked function module (6, 7).

2. Surgical lamp according to claim 1, wherein several identical first interfaces are provided at the lamp body (1).

3. Surgical lamp according to claim 2, wherein several function modules (6, 7) are provided.

4. Surgical lamp according to claim 2 or 3, wherein the first interfaces are arranged at the outer area of the lamp body (1).

5. Surgical lamp according to anyone of the preceding claims, wherein the first interface and the second interface are configured such that their connecting and separating is made without tools.

6. Surgical lamp according to anyone of the preceding claims, wherein the control device (4) comprises a memory area (22), and, in the memory area (22), various illumination scenarios are stored.

7. Surgical lamp according to claim 6, wherein at least one of the function modules (6, 7) comprises a lighting module.

8. Surgical lamp according to claim 7, wherein illumination scenarios comprise data ensuring the adherence of the limit value for a maximum illuminance in the surgical field depending on the kind and/or the number of the lighting modules.

9. Surgical lamp according to anyone of claims 6 to 8, wherein at least one of the function modules (6, 7) comprises a sensor module.

10. Surgical lamp according to claim 9, wherein the sensor module is configured to give data concerning a position of an obstacle present between the lamp body (1) and the surgical field to the control device (4), and an illumination scenario, according to which the illuminants (3) are controlled depending on the position of the obstacle such that the illuminant (3) having a light ray in which the obstacle is located is dimmed or switched off, is stored.

11. Surgical lamp according to anyone of the preceding claims, wherein the at least one function module (6, 7) comprises a memory element (18) for storing operating data, and
wherein the control device (4) is configured to retrieve the operating data from the memory element (18) of the at least one function module (6, 7) and to control the at least one function module (6, 7) as well as the lamp body (1) according to the retrieved operating data.

12. Surgical lamp according to claim 11, wherein the control device (4) is configured, after each connecting of the first interface and second interface, to retrieve the operating data from the memory area of the connected function module and to control the surgical lamp according to the retrieved operating data.

13. Surgical lamp according to claim 11 or 12, wherein the stored operating data comprise both operating parameter and operating software.

14. Surgical lamp according to claim 13, wherein the control device (4) is configured to recognize whether a function module (6, 7) having a predetermined function not being stored so in the control device (4) is connected for the first time to the control device (4) and to perform an update of the operating software of the control device (4) according to the operating software stored in the function module (6, 7) .

15. Surgical lamp according to any one of the claims 11 to 14, wherein the control device (4) is configured to control a specific function module (6, 7) according to operating data retrieved from another than the specific function module (6, 7) .

16. Surgical lamp according to anyone of the preceding claims, wherein the lamp body (1) comprises at least one notch (10) for attaching the function modules (6, 7) and a cover (8), and the at least one notch (10) is coverable by the cover (8) when no function module (6, 7) is attached.

17. Surgical lamp according to anyone of the preceding claims, wherein the function modules (6, 7) comprise an activation device (21) and the lamp body (1) comprises a switching device (20) switched by the activation device (21) for each first interface so that the power supply terminal (12) of the first interface is connected to a power supply unit (17) and/or the data connection terminal (13) of the first interface is connected to the control device (4) when the function module (6, 7) is connected to the lamp body (1) via the first interface and the second interface, and the power supply terminal (12) of the first interface is separated from the power supply unit (17) and/or the data connection terminal (13) of the first interface when the function module (6, 7) is not fixed to the lamp body (1) via the first interface and the second interface.

18. Method for operating a surgical lamp according to anyone of the claims 1 to 17, having the steps:
- connecting the second interface of the at least one function module (6, 7) to the first interface of the lamp body (1);
**characterized by**
- recognizing the type of the docked function module (6, 7)
- controlling the at least one illuminant (3) depending on the type of the docked function module (6, 7).

19. Method according to claim 18 with a surgical lamp according to claim 6, wherein the at least one illuminant (3) is controlled depending on the type of the docked function module (6, 7) according to the stored illumination scenario.

20. Method according to claim 18 or 19 with a surgical lamp according to claim 11 having the additional steps:
- retrieving the operating data from the memory element (18) of the at least one function module (6, 7);
- controlling the function module (6, 7) as well as the lamp body (1) according to the retrieved operating data.

21. Method according to anyone of the claims 18 to 20 with the step:
- controlling the function module (6, 7) according to the operating data retrieved from one of the other function modules.

22. Method according to anyone of the claims 18 to 21 with a surgical lamp according to claim 17 with the additional step:
- switching a switching device (20) by means of an activation device (21) and, thereby, connecting the power supply terminal (12) of the first interface to the power supply unit (17) and connecting the data connection device (13) of the first interface to the control device (4).

23. Method according to anyone of the claims 20 to 22 with a surgical lamp according to claim 15 with the additional step:
- controlling one of the function modules (6, 7) according to the operating data retrieved from one of the other function modules (6, 7).

## Revendications

1. Scialytique destiné à éclairer un champ opératoire sur un corps humain
comprenant un corps de scialytique (1),
un dispositif de commande (4), et
au moins un module fonctionnel (6, 7) séparé,
dans lequel
le corps de scialytique (1) présente au moins un moyen d'éclairage (3) destiné à éclairer le champ opératoire, et
au moins une première interface pourvue d'un premier dispositif de fixation (11), d'un raccord d'alimentation électrique (12) et d'une liaison de données (13),
le dispositif de commande (4) est prévu pour commander ledit au moins un moyen d'éclairage (3) et est connecté à la liaison de données (13) de ladite au moins une première interface aux fins de transmission de données, et
ledit au moins un module fonctionnel (6, 7) présente une deuxième interface pourvue d'un deuxième dispositif de fixation (14), d'un deuxième raccord d'alimentation électrique (15) et/ou d'une deuxième liaison de données (16), qui peut être arrimée à la première interface, **caractérisé en ce que**
le corps de scialytique (1) comprend un dispositif d'identification destiné à identifier un type d'un module fonctionnel (6, 7) arrimé, et
le dispositif de commande (4) est adapté à commander ledit au moins un moyen d'éclairage (3) dans le corps de scialytique (1) en fonction du type du module fonctionnel (6, 7) arrimé.

2. Scialytique selon la revendication 1, dans lequel plusieurs premières interfaces identiques sont prévues sur le corps de scialytique (1).

3. Scialytique selon la revendication 2, dans lequel plusieurs modules fonctionnels (6, 7) sont prévus.

4. Scialytique selon la revendication 2 ou 3, dans lequel les premières interfaces sont agencées dans la zone extérieure du corps de scialytique (1).

5. Scialytique selon l'une quelconque des revendications précédentes, dans lequel la première interface et la deuxième interface sont réalisées de telle manière que leur liaison et leur séparation s'effectuent sans outil.

6. Scialytique selon l'une quelconque des revendications précédentes, dans lequel le dispositif de commande (4) comprend une zone de mémoire (22), et différents scénarios d'éclairage sont mis en mémoire dans la zone de mémoire (22).

7. Scialytique selon la revendication 6, dans lequel au moins un des modules fonctionnels (6, 7) comprend un module lumineux.

8. Scialytique selon la revendication 7, dans lequel les scénarios d'éclairage présentent des données qui garantissent le respect d'une valeur limite pour un éclairement maximal dans le champ opératoire en fonction du type et/ou du nombre de modules d'éclairage.

9. Scialytique selon l'une quelconque des revendications 6 à 8, dans lequel au moins un des modules fonctionnels (6, 7) comprend un module de capteur.

10. Scialytique selon la revendication 9, dans lequel le module de capteur est adapté à transmettre au dispositif de commande (4) des données concernant une position d'un obstacle présent entre le corps de scialytique (1) et le champ opératoire, et un scénario d'éclairage est mis en mémoire, selon lequel les moyens d'éclairage (3) sont commandés en fonction de la position de l'obstacle, de sorte que l'intensité des moyens d'éclairage (3) présentant un faisceau lumineux dans lequel se situe l'obstacle est atténuée ou que lesdits moyens sont mis hors tension.

11. Scialytique selon l'une quelconque des revendications précédentes, dans lequel ledit au moins un module fonctionnel (6, 7) comprend un élément de mémoire (18) pour la mise en mémoire de données de fonctionnement, et
dans lequel le dispositif de commande (4) est adapté à consulter les données de fonctionnement dans l'élément de mémoire (18) dudit au moins un module fonctionnel (6, 7) et à commander aussi bien ledit au moins un module fonctionnel (6, 7) que le corps de scialytique (1) conformément aux données de fonctionnement consultées.

12. Scialytique selon la revendication 11, dans lequel le dispositif de commande (4) est adapté à consulter les données de fonctionnement dans la zone de mémoire du module fonctionnel connecté et à commander le scialytique conformément aux données de fonctionnement consultées, une fois la première interface et la deuxième interface connectées.

13. Scialytique selon la revendication 11 ou 12, dans lequel les données de fonctionnement mises en mémoire présentent aussi bien des paramètres de fonctionnement qu'un logiciel de fonctionnement.

14. Scialytique selon la revendication 13, dans lequel le dispositif de commande (4) est adapté à identifier si un module fonctionnel (6, 7) présentant une fonction prédéfinie non mise en mémoire dans le dispositif de commande (4) est connecté pour la première fois au dispositif de commande (4), et à mettre en oeuvre une mise à jour du logiciel de fonctionnement du dispositif de commande (4) conformément au logiciel de fonctionnement mis en mémoire dans le module fonctionnel (6, 7).

15. Scialytique selon l'une quelconque des revendications 11 à 14, dans lequel le dispositif de commande (4) est adapté à commander un module fonctionnel (6, 7) conformément aux données de fonctionnement consultées dans un module fonctionnel différent du module fonctionnel (6, 7) défini.

16. Scialytique selon l'une quelconque des revendications précédentes, dans lequel le corps de scialytique (1) comprend au moins un évidement (10) pour le montage des modules fonctionnels (6, 7) et un couvercle (8), et ledit au moins un évidement (10) peut être recouvert par le couvercle (8), lorsqu'aucun module fonctionnel (6, 7) n'est monté.

17. Scialytique selon l'une quelconque des revendications précédentes, dans lequel les modules fonctionnels (6, 7) comprennent un dispositif d'activation (21) et le corps de scialytique (1) présente pour chaque première interface un dispositif de commutation (20), qui est mis en tension par le dispositif d'activation (21), de sorte que le raccord d'alimentation d'électrique (12) de la première interface est connecté à une unité d'alimentation électrique (17) et/ou que le raccord de liaison de données (13) de la première interface est connecté au dispositif de commande (4), lorsque le module fonctionnel (6, 7) est fixé au corps de scialytique (1) par l'intermédiaire de la première interface et de la deuxième interface, et que le raccord d'alimentation électrique (12) de la première interface est séparé de l'unité d'alimentation en courant (17) et/ou que le raccord de liaison de données (13) de la première interface est séparé du dispositif de commande (4), lorsque le module fonctionnel (6, 7) n'est pas fixé au corps de scialytique (1) par l'intermédiaire de la première interface et de la deuxième interface.

18. Procédé de fonctionnement d'un scialytique selon l'une quelconque des revendications 1 à 17, comprenant les étapes
- de connexion de la deuxième interface dudit au moins un module fonctionnel (6, 7) à la première interface du corps de scialytique (1) ;
**caractérisé par** :
- l'identification du type du module fonctionnel (6, 7) arrimé ;
- la commande dudit au moins un moyen d'éclairage (3) en fonction du type du module fonctionnel (6, 7) arrimé.

19. Procédé selon la revendication 18 comprenant un scialytique selon la revendication 6, dans lequel ledit au moins un moyen d'éclairage (3) est commandé en fonction du type du module fonctionnel (6, 7) arrimé conformément à un scénario d'éclairage mis en mémoire.

20. Procédé selon la revendication 18 ou 19 comprenant un scialytique selon la revendication 11, comprenant les étapes supplémentaires
- de consultation des données de fonctionnement dans l'élément de mémoire (18) dudit au moins un module fonctionnel (6, 7) ;
- de commande aussi bien du module fonctionnel (6, 7) que du corps de scialytique (1) conformément aux données de fonctionnement consultées.

21. Procédé selon l'une quelconque des revendications 18 à 20 comprenant l'étape
- de commande du module fonctionnel (6, 7) conformément aux données de fonctionnement consultées dans un module fonctionnel différent des autres modules fonctionnels (6, 7).

22. Procédé selon l'une quelconque des revendications 18 à 21 comprenant un scialytique selon la revendication 17, comprenant l'étape supplémentaire
- de mise en marche d'un dispositif de commutation (20) au moyen d'un dispositif d'activation (21) et de connexion du raccord d'alimentation électrique (12) de la première interface à l'unité d'alimentation électrique (17) et de connexion de la liaison de données (13) de la première interface au dispositif de commande (4).

23. Procédé selon l'une quelconque des revendications 20 à 22 comprenant un scialytique selon la revendication 15, comprenant l'étape supplémentaire
- de commande d'un des modules fonctionnels (6, 7) conformément aux données de fonctionnement consultées dans un module fonctionnel différent des autres modules fonctionnels (6, 7) .
